# EUROPEAN PATENT APPLICATION

(11) **EP 1 992 285 A1**
(43) Date of publication of application: **19.11.2008**
(21) Application number: 07738013.7
(22) Date of filing: 08.03.2007
(51) Int. Cl.: A61B 5/151

(54) **LANCET DEVICE**

(30) Priority: 08.03.2006 JP 2006062356
(71) Applicant: Arkray, Inc., Kyoto-shi, Kyoto 601-8045 (JP)
(72) Inventor: FUKUZAWA, Masahiro, Kyoto-shi, Kyoto 601-8045 (JP); NISHIYAMA, Hisashi, Kyoto-shi, Kyoto 601-8045 (JP)
(74) Representative: Kastel, Stefan
(86) International application number: PCT/JP2007/054522
(87) International publication number: WO 2007/102576

(57) **Abstract**

In the lancet device (10) of the present embodiment, the puncture needle (21) is received the biasing force applied by the coil spring (31)a and is projected toward the tip side, and is retracted by the biasing force applied by the return spring (31b). In the lancet device (10), the colliding part (32b) and the collision receiving part (38a) are made to collide when the puncture needle (21) is projected, and the abutting stopping member (38) elastically deforms. The colliding part (32b) is a part of the lancet holder (32) that holds the lancet (20) having the puncture needle (21). The collision receiving part (38a) is a part of the abutting stopping member (38). The contact part (38b) of the abutting stopping member (38) moves in the direction of the colliding part (32b) and comes into contact with the colliding part (32b) by the elastic deformation of this abutting stopping member (38), and the force that retracts the lancet holder (32) is weakened.

## Description

### TECHNICAL FIELD

The present invention relates to a lancet device (puncture device) that is retracted after a lancet having a puncture needle is projected by an internally housed spring or the like in the puncture direction and forms a puncture wound in the skin of a fingertip or the like.

### BACKGROUND ART

With the increase in diabetes patients in recent years, there has been an increase in diabetes patients measuring their own blood sugar levels at home or the like and managing the changes in their blood sugar levels themselves. In light of this situation, lancet devices (puncture devices) equipped with puncture needles capable of easily creating a wound in a fingertip or the like and collecting the required blood for the measurements have been provided when blood is collected for measuring the blood sugar level.
The lancet device is provided with a lancet holder that holds a lancet that has a puncture needle on its tip part, and when the tip part is placed against a fingertip or the like the puncture needle is projected with the lancet holder using the force of a spring or the like, projecting the needle approximately several tenths of a mm to 2.0 mm from the tip. Thus, an incision is made in the fingertip or the like, and the blood that flows from the puncture wound is collected. The blood sugar level may be measured by dripping it on a part such as a sensor part of a blood sugar tester.
This lancet device incorporates two springs (biasing means), a forward drive spring that applies biasing force for projecting the puncture needle forward in the puncture direction and a retraction spring that applies biasing force in a direction that retracts the puncture needle that has been projected into the fingertip. Furthermore, the spring used for the retraction spring uses a spring that applies biasing force that is smaller than that of the forward drive spring. Therefore, there is not much effect on the biasing force applied for forward drive when the puncture needle is projected, and the puncture needle may be retracted after being projected by the biasing force applied by the retraction spring.
However, after the puncture needle has first punctured the skin one time in this lancet device, the puncture may be carried out a second or more times even though it has been retracted by the biasing force applied by the retraction spring that pulls it back. These multiple punctures are brought about because energy is saved up with the compression of the forward drive spring by the biasing force retracting the puncture needle caused by the biasing force applied by the retraction spring that moves it back, and the biasing force applied for driving the puncture needle forward is generated once again. In other words, in conventional lancet devices, the puncture needle is repeatedly driven forward and retracted because the biasing means such as the two springs and the like is repeated with the compression and elongation after the puncture needle is projected, and this action is gradually dampened and stops.
Therefore, after a puncture wound has been formed by the first puncture, unnecessary puncture wounds are formed by the second and later punctures, and there is unnecessary suffering by the user of the lancet device.
Conversely, Patent References 1 and 2 disclose constitutions for preventing multiple punctures by the puncture needle of this type of a lancet device.
For example, Patent Reference 1 discloses a lancet device capable of preventing multiple punctures by the puncture needle where a brake is applied through the force of friction in the lancet holder that holds the puncture needle. Specifically, the lancet holder that holds the puncture needle generates frictional force between it and a brake member and undergoes braking by coming into contact with the brake member on the outside thereof when moving forward in the puncture direction. Thus, the amplitude of the forward and retracting movement of the lancet holder that holds the puncture needle is dampened after the puncture, and multiple punctures may be prevented.
[Patent Reference 1]
   Published Unexamined Patent Application No. 2000-237172 (published September 5, 2000)
[Patent Reference 2]
   Published Unexamined Patent Application No. 2000-166902 (published June 20, 2000)
[Patent Reference 3]
   Published Unexamined Patent Application No. 11-76211 (published March 23, 1999)

### SUMMARY OF THE INVENTION

However, the conventional lancet device described above has the following problems.
Namely, the lancet devices disclosed in the publications described above have a mechanism where the outside surface of the lancet holder comes into contact with a brake member during the puncture and a frictional force is generated. Therefore, the size of the frictional force necessary to prevent multiple punctures greatly depends on the dimensional precision of the outside of the lancet holder, the brake member and the like. In addition, since these lancet devices are typically made of resin, it is easy for the dimensions to change because of linear expansion caused by variations in temperature. Therefore, there is a risk that a frictional force that is greater than necessary and prevents projection of the puncture needle may arise or that frictional force sufficient for avoiding multiple punctures may not be obtained. As a result, high dimensional precision is required, and strict inspections must be carried out after assembly.
The problem for the present invention is providing a lancet device capable of avoiding multiple punctures by the puncture needle stably even if strict management of dimensional precision is not carried out.
The lancet device according to the first aspect of the present invention is provided with a lancet holder, a first biasing part, a second biasing part and an abutting stopping member. In addition to the lancet, which includes the puncture needle, being attached from the front side in the puncture direction, the lancet holder has a colliding part that includes a surface that intersects the puncture direction. The first biasing part applies a force that moves the lancet holder forward in the puncture direction. The second biasing part retracts the lancet holder that has been moved forward by applying a force that is smaller than the force applied by the first biasing part. The abutting stopping member has a collision receiving part and a contact part. When the lancet holder has force applied by the first biasing part and moves forward in the puncture direction, the collision receiving part collides with the colliding part and limits the forward motion of the lancet holder in the puncture direction. The contact part comes into contact with the colliding part due to elastic deformation that arises when the colliding part and the collision receiving part collide.
When a part (colliding part) of the lancet holder and a part (collision receiving part) of the abutting stopping member collide, the abutting stopping member receives the energy and is deformed elastically. Then the contact part which is part of the abutting stopping member comes into contact with the colliding part of the lancet holder by the energy, and the retraction force of the lancet holder is weakened.
Here, the first biasing part and the second biasing part are, for example, elastic members such as springs, and each of them stores energy when compressed, moving the puncture needle along with the lancet holder in the puncture direction.
Normally, in the lancet devices provided with a first biasing part that projects the puncture needle and a second biasing part that retracts the puncture needle after the puncture in this manner, the puncture needle that has been projected in the forward puncture direction by the elastic force of the first biasing part is retracted by the elastic force of the second biasing part. At this time, the energy when the puncture needle is retracted by the second biasing part compresses the first biasing part, and as a result of energy accumulating in the first biasing part the puncture needle is once again moved forward by the energy from the first biasing part and multiple punctures may occur. In this case, unnecessary puncture wounds are formed in the user, and there is unnecessary suffering, which is not preferable.
Therefore, when the puncture needle is projected by the first biasing part in the lancet device of the present invention, the abutting stopping member is elastically deformed by the energy when the lancet holder that holds the puncture needle and the abutting stopping member come into contact, the contact part which is a part thereof, is made to contact the colliding part which is part of the lancet holder.
Thus, with the lancet holder that has first collided with the abutting stopping member, a part (colliding part) thereof is in a state where it is held tachistoscopically by the contact part of the abutting stopping member. Therefore, the force of the retraction by the second biasing part with the repelling force of the energy when the puncture needle is projected in the present lancet holder is moderated by the contact with the contact part. Thus, by weakening the force of the retraction of the lancet holder by elastic deformation of the abutting stopping member, the number and the amplitude of back and forth movements of the lancet holder that includes the puncture needle that are repeated after the puncture needle is projected are reduced, and multiple punctures may be prevented if there is no requirement for high dimensional precision for the lancet holder and the other members as with the conventional.
Specifically, when the reduction in the force of the lancet holder retraction in the present invention is carried out using the collision energy between the lancet holder and the contact part, it may be assumed that the greater the collision energy is the greater the extent of the elastic deformation of the abutting stopping member is. Therefore, when the collision energy is large, that is, the larger the possibility of multiple punctures with a large force that retracts the lancet holder is, the greater the intensity of the contact of the contact part with the colliding part of the lancet holder becomes, and the time increases. As a result, the greater the collision energy becomes, the greater the force that weakens the force of the lancet holder retraction may be made, and multiple punctures by the puncture needle may be prevented effectively after projection.
The lancet device according to second aspect of the invention is the lancet device of the first aspect of the invention wherein the contact part of the abutting stopping member has a first contact part and a second contact part disposed so as to be opposed to each other along the puncture direction. Furthermore, when the colliding part passes between the first contact part and the second contact part and collides with the collision receiving part, the abutting stopping member elastically deforms, and the first contact part and the second contact part sandwich the side surfaces of the colliding part.
Here, the side surfaces of the colliding part that has passed between the first contact part and the second contact part and collided with the collision receiving part are in contact so as to be sandwiched between the first contact part and second contact part, which are included on the abutting stopping member that is elastically deformed by the collision.
Thus, the energy at the time of the collision of the colliding part and the collision receiving part is converted into energy for elastically deforming the abutting stopping member to weaken the force for retracting the lancet holder. As a result, even though it is a simple constitution, the amplitude of the back-and-forth movement of the lancet holder because of the first biasing part and the second biasing part is reduced, and the occurrence of multiple punctures may be prevented.
The lancet device according to the third aspect of the invention is the lancet device of the first or second aspect of the invention wherein an elastic body is provided between the colliding part and collision receiving part.
Here, the elastic body is disposed between the colliding part of the lancet holder and the collision receiving part of the abutting stopping member, that is, on the side of the colliding part of the lancet holder or the side of the collision receiving part of the abutting stopping member.
Thus, when the colliding part and the collision receiving part collide, the two collide through the elastic body, so the energy at the time of collision is absorbed by the elastic body, and a longer collision time may be assured. Therefore, the time for the elastic deformation of the abutting stopping member is increased, and the time that the contact part is in contact with the colliding part is increased. As a result, the retraction force for the colliding part of the lancet holder is effectively weakened, and the occurrence of multiple punctures may be avoided.
The lancet device according to fourth aspect of the invention is the lancet device according to any one of the first through third aspects of the invention wherein the first biasing part and the second biasing part are coil springs.
Here, coil springs are used for the first biasing part that projects the puncture needle along with the lancet holder forward in the puncture direction and the second biasing part that retracts the puncture needle after projection.
Thus, multiple punctures by the puncture needle may effectively be prevented because the contact part of the abutting stopping member comes into contact with the colliding part of the lancet holder and weakens the force during retraction when back-and-forth movement of the puncture needle (lancet holder) in the puncture direction occurs after projection because of the two coil springs.
The lancet device according to the fifth aspect of the present invention is provided with a lancet holder, a first force applying member, a second force applying member and a pressing pressure part. The lancet holder has an elastic member that elastically deform in a direction that intersects the puncture direction and includes a sloped surface sloped with respect to the puncture direction. The first biasing part applies a force that moves the lancet holder forward in the puncture direction. The second biasing part retracts the lancet holder that has been moved forward by a force that is smaller than the force applied by the first biasing part. When the lancet holder has moved to the vicinity of a position for a state where the lancet can be projected by the first biasing part, the pressing pressure part makes contact with the sloped surface on the elastic member, and the elastic member is pressed in a direction that intersects the puncture direction.
For example, the elastic member that elastically deforms in the direction intersecting the puncture direction is provided on part of the lancet holder when the lancet holder moves towards the vicinity of a projection pause position and comes in contact with the pressing pressure part formed in part of the side of the case.
Here, the first biasing part and the second biasing part are, for example, elastic members such as springs, and each of them stores energy when compressed, moving the puncture needle along with the lancet holder in the puncture direction.
Normally, in lancet devices provided with a first biasing part that projects the puncture needle and a second biasing part that retracts the puncture needle after the puncture in this manner, the puncture needle that has been projected in the forward puncture direction by the elastic force of the first biasing part is retracted by the elastic force of the second biasing part. At this time, the energy when the puncture needle is retracted by the second biasing part compresses the first biasing part, and as a result of energy accumulating in the first biasing part, the puncture needle is once again moved forward by the energy from the first biasing part and multiple punctures may occur. In this case, unnecessary puncture wounds are formed in the user, and there is unnecessary suffering, which is not preferable.
With the lancet device of the present invention, the elastic member is made to elastically deform in the direction intersecting the puncture direction by, for example, with the sloped surface of the elastic member, which is part of the lancet holder, coming into contact with the pressing pressure part formed on the case side or the like in the vicinity of the projection pause position of the lancet holder.
Thus, part of the energy of movement in the puncture direction for the lancet holder, which receives the spring force of the first and second biasing parts and moves back and forth in a fixed range, is converted to energy that elastically deforms the elastic member in the direction intersecting the puncture direction, and braking may be applied to the movement of the lancet holder. As a result, the risk of multiple punctures after the puncture because of the back-and-forth movement of the lancet holder between the projection pause position and puncture position caused by the elastic force from the first and second biasing parts may be avoided.
In addition, when the lancet holder that has been projected toward the puncture direction once is returned in the opposite direction by the elastic energy from the second biasing part, the elastic energy stored in the first biasing part for moving the lancet holder in the puncture direction again may be reduced by providing the contact position for this elastic member and the pressing pressure part in the vicinity of the projection pause position for the lancet holder.
Furthermore, the braking force applied to the lancet holder is generated by contact between the elastic member and the pressing pressure part, so dependence on dimensional precision may be reduced and product yields may be increased over the conventional constitution where braking is applied to the lancet holder by frictional force.
From the above, the risk of the so-called second stab may be avoided, and a lancet device with a higher level of safety may be provided.
The lancet device according to the sixth aspect of the invention is provided with a lancet holder, first force applying member, second force applying member and pressing pressure part. The lancet holder has an elastic member that is elastically deformed in the direction intersecting the puncture direction and includes a first sloped surface sloped with respect to the puncture direction and a second sloped surface for which the angle of the slope is greater than the first sloped surface. The first biasing part applies a force that moves the lancet holder forward in the puncture direction. The second biasing part retracts the lancet holder that has been driven forward by a force that is smaller than the force applied by the first biasing part. The pressing pressure part comes into contact with the first sloped surface and the second sloped surface of the elastic member and presses the elastic member in the direction intersecting the puncture direction.
Here for example, the elastic member that elastically deforms in the direction intersecting the puncture direction because of the pressing pressure member formed in the case side or the like has the first sloped surface sloped with respect to the puncture direction and the second sloped surface for which the angle of the slope is greater than the first sloped surface.
Thus, when the pressing pressure part has come into contact with the first sloped surface, the amount of braking force applied to the lancet holder may be changed when it comes into contact with the second sloped surface. Specifically, since more energy is necessary for elastic deformation of the elastic member when the second sloped surface, which has a larger angle for the slope comes into contact, a stronger braking force may be applied to the lancet holder when contact is made with the second sloped surface than when contact is made with the first sloped surface. As a result, a braking force of a suitable strength may be applied according to the direction the lancet holder is moving, so the risk of the so-called second stab after the puncture may be avoided effectively and a lancet device with a higher level of safety may be provided.
The lancet device according to the seventh aspect of the invention is the lancet device according to the sixth aspect of the invention wherein the first sloped surface is formed in a forward orientation with respect to the puncture direction and the second sloped surface is formed in a backward orientation with respect to the puncture direction so as to be in contact adjacent to the first sloped surface.
Here, the first sloped surface and the second sloped surface are formed in a forward orientation and backward orientation, respectively, with respect to the puncture direction.
Thus, when the lancet holder after puncture moves back and forth because of the elastic energy from the first and second biasing parts, a greater braking force may be applied to the lancet holder when it moves backwards than when it moves forward. As a result, the lancet holder is applied no large braking force when the lancet holder is projected for making the puncture. On the other hand, the lancet holder is applied large braking force when the lancet holder is retracted after the puncture. Thus, braking force may be applied to the lancet holder effectively after the puncture has been carried out smoothly, so the risk of a second stab occurring after the puncture may be avoided.
The lancet device according to the eighth aspect of the invention is the lancet device according to the sixth or seventh aspect of the invention wherein the pressing pressure part is in contact with the first sloped surface and the second sloped surface when the lancet holder has moved into the vicinity of the position where it may be projected by the first biasing part.
Here, the vicinity of the projection pause position for the lancet holder is set as the position where the pressing pressure part is in contact with the first and second sloped surfaces.
Thus, when the lancet holder that has been projected in the puncture direction once returns in the opposite direction because of the elastic energy from the second biasing part, the elastic energy that is stored in the first biasing part for moving the lancet holder in the puncture direction once again may be reduced. As a result, the risk of the so-called second stab may be avoided, and a lancet device with a high level of safety may be provided.
The lancet device according to the ninth aspect of the invention is the lancet device according to any one of the fifth through eighth aspects of the invention wherein the pressing pressure part is a rib member that is formed in the case part inside which the lancet holder is inserted and extends in a direction intersecting the puncture direction.
Here, a rib member formed in the side of the case part is used as the pressing pressure part.
Thus, in the range of movement of the lancet holder from the projection pause position to the puncture position, pressing pressure is applied to the elastic member by the simple constitution of the rib member formed in the case part, and a braking force may be effectively applied to the lancet holder.
The lancet device according to the tenth aspect of the invention is the lancet device according to any one of the fifth through eighth aspects of the invention wherein the pressing pressure part includes puncture depth adjusting member that adjusts the puncture depth of the puncture needle attached to the tip of the lancet holder.
Thus, multiple punctures may easily be prevented even if there is a constitution where it is difficult to provide the pressing pressure part, such as ribs, on the inside walls of the case part or the like in which the lancet holder is housed by providing the pressing pressure part on part of an existing rotating type depth adjusting member provided on the back end or the like of the case part. In addition, increased costs and reductions in ease of assembly because of increases in the number of parts may be avoided.
The lancet device according to the eleventh aspect of the invention is the lancet device according to any one of the fifth through eighth aspects of the invention wherein the lancet holder has a contact part that comes into contact with the end part of the second biasing part when the lancet holder is moved forward in the puncture direction.
Thus, the occurrence of multiple punctures may be avoided while the lancet holder is being retracted by making the contact part formed in part of the lancet holder come into contact with the second biasing part, such as a return spring or the like, during the puncture.

### BRIEF DESCRIPTION OF DRAWINGS

Fig. 1 is a perspective view is showing the appearance of a lancet device according to a first embodiment of the present invention.
Fig. 2 is a perspective view showing the lancet and the main body constituting the lancet device in Fig. 1.
Fig. 3 is a partial cross-sectional view showing the constitution of the inside of the casing provided by the lancet device in Fig. 1.
Fig. 4 is a perspective view showing the puncture body and cap provided by the lancet in Fig. 2.
Fig. 5 is a cross-sectional view showing the constitution of the inside of the lancet device.
Fig. 6 is a partially enlarged side cross-sectional view of the constitution of part of the abutting stopping member provided by the lancet device in Fig. 1.
Fig. 7 is a perspective view showing the constitution of the back end side of the lancet holder provided by the lancet device in Fig. 1.
Fig. 8 (a) to (c) are side cross-sectional views showing the positional relationships of the lancet holder and the abutting stopping member when the puncture is performed.
Fig. 9 is a cross-sectional view showing the constitution of the inside of the lancet device according to another embodiment of the present invention.
Fig. 10 is an enlarged view of part A in Fig. 9.
Fig. 11 (a) to (d) are partial cross-sectional views showing the movement of the lancet holder at the time the lancet is set, during puncture and after puncture.
Fig. 12 is a cross-sectional view showing the constitution of the inside of the lancet device according to yet another embodiment of the present invention.
Fig. 13 (a) is a cross-sectional view at line A showing part of a disengaging part included in the lancet device in Fig. 12. (b) is a cross-sectional view at line B showing the shapes of the back end parts of the lancet holder that pass through the opening therein.
Fig. 14 (a) to (c) are cross-sectional views of line C showing the states of the lancet holder in Fig. 12 before puncture, during puncture and after puncture.
Fig. 15 is a cross-sectional view showing the constitution of the inside of the lancet device according to yet another embodiment of the present invention.
Fig. 16 (a) and (b) are enlarged cross-sectional diagrams showing the states of the back end part side of the lancet device in Fig. 15 before and after puncture, respectively. EXPLANATION OF REFERENCE

- 10: lancet device (puncture device)
- 20: lancet
- 21: puncture needle
- 22: casing
- 22a: inner peripheral surface
- 22b: convex portion
- 22c: groove
- 23: puncture body
- 23a: tapered part
- 23b: flange part
- 23c: groove
- 23d: insertion part
- 23e: groove
- 24: gap
- 24a: raised part
- 24b: cover part
- 24c: hole
- 30: main body
- 31a: coil spring (first biasing part)
- 31b: return spring (second biasing part)
- 32: lancet holder
- 32a: convex portion
- 32b: colliding part
- 32c: notched part
- 33: rotating body (puncture depth adjusting member)
- 33a: rib
- 33b: rib (pressing pressure part)
- 34: biasing part
- 35: housing
- 35a: puncture opening
- 35b: opening
- 35c: notched part
- 36: disengaging part
- 36a: wall part
- 36aa: opening
- 37: setting release button
- 38: abutting stopping member
- 38a: collision receiving part
- 38b: contact part (first contact part, second contact part)
- 38c: base
- 38d: screw indentation
- 39: elastic part (elastic body)
- 50: lancet device (puncture device)
- 60: lancet
- 70: main body
- 72: lancet holder
- 72b: colliding part
- 72d: elastic member
- 72da: first sloped surface
- 72db: second sloped surface
- 72e: rib (contact part)
- 78: abutting stopping member
- 784a: raised part
- 78d: collision receiving part
- 80: rib member (pressing pressure part)
- 150: lancet device (puncture device)
- 170: main body
- 250: lancet device (puncture device)
- 270: main body

### DETAILED DESCRIPTION OF THE PREFERRED EMBODIMENTS

### <Embodiment 1>

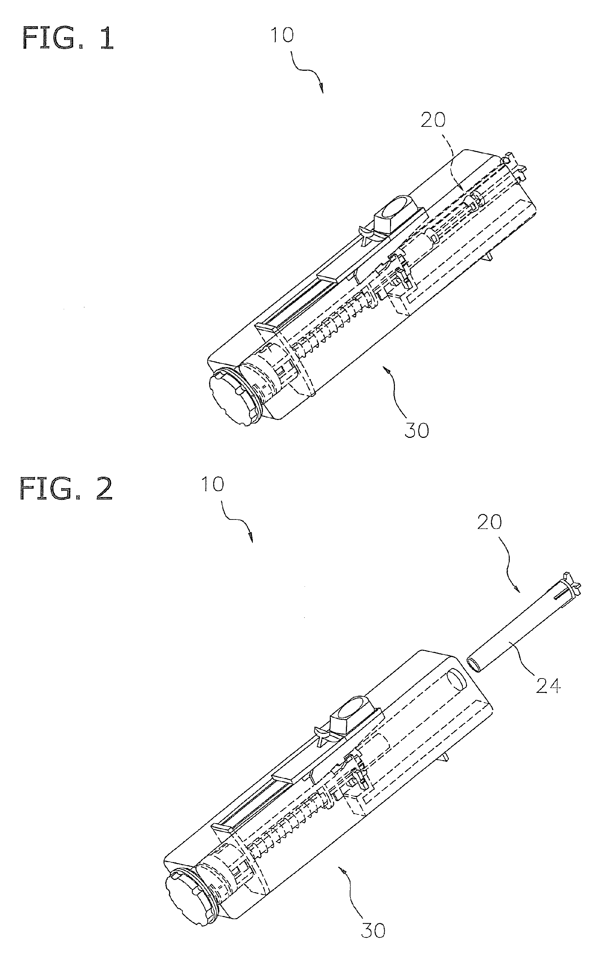

The lancet device according to an embodiment of the present invention is described as follows using Fig. 1 through Fig. 8(c).

### <Constitution of the lancet device 10 >

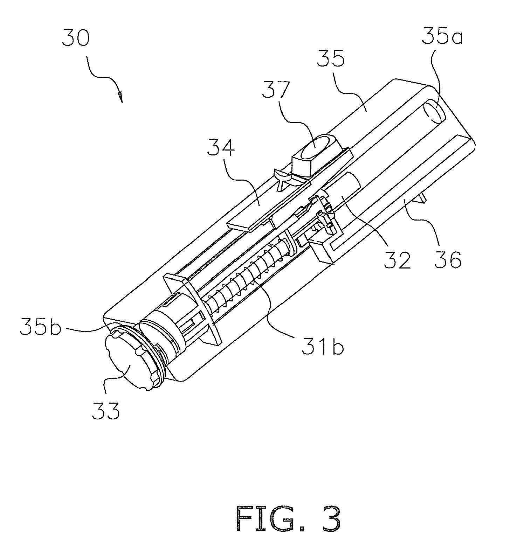

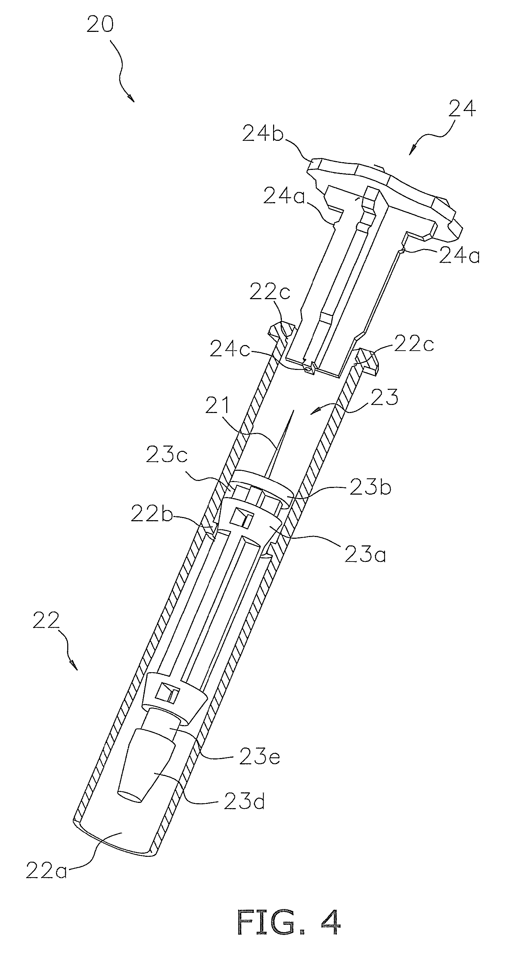

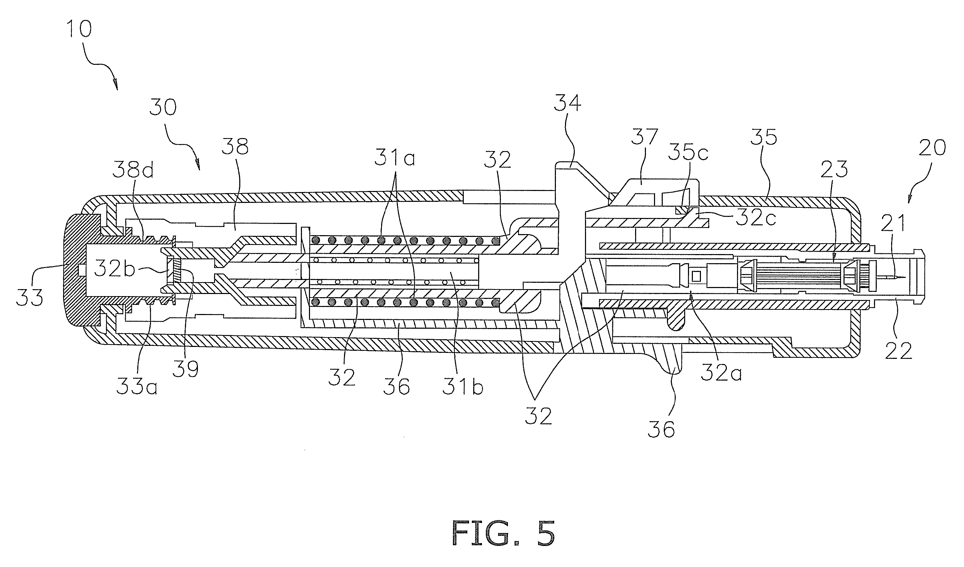

The lancet device (puncture device) 10 according to the present embodiment is a device that is used for collecting body fluids when diabetics measure their blood sugar level and the like. When it is used, a puncture needle 21 (see Fig. 4) is made to protrude from and opening formed in the tip part in a state where the tip part is in contact with the skin and a puncture wound is formed.
Specifically, the lancet device 10 is provided with a lancet 20 and a main body 30 as is shown in Fig. 1 and Fig. 2.
The lancet 20 has the puncture needle 21 (see Fig. 4) for forming the puncture wound inside, and as is shown in Fig. 2 it is attached from the tip side of the main body 30.
The main body 30 incorporates a coil spring (first biasing part) 31a (see Fig. 5) that applies force to the puncture needle 21 (see Fig. 4) for making it protrude in a prescribed puncture direction and a return spring (second biasing part) 31b (see Fig. 3 and Fig. 5) that returns the puncture needle 21 that has been projected by the coil spring 31a into the housing 35.
Moreover, the "tip side" used in the description hereinafter is the side where the tip of the puncture needle 21 of the lancet 20, which will be described below, protrudes and the "back end side" is the side opposite to this.

### <Constitution of the lancet 20>

The lancet 20, as is shown in Fig. 4, is provided with a substantially cylindrical casing 22 and a puncture body 23 housed in a movable state to the tip side and the back end side in the puncture direction within the casing 22 when the lancet device 10 is used. Moreover, in Fig. 4, a cross-sectional view of the casing 22 is shown for convenience in describing the inside of the casing 22 having a substantially cylindrical shape.
As is shown in Fig. 4, the puncture body 23 is made from resign and formed as a unit with the puncture needle 21 for forming the puncture wound in the skin. In addition, the part formed of resin in the puncture body 23 has a tapered part 23a, a flange part 23b, a groove 23c, an insertion part 23d and a groove 23e formed. The tapered part 23a, flange part 23b and groove 23c, are formed in the tip side where the puncture needle 21 protrudes. The tapered part 23a is a member that gets thinner toward the back end side, and the cross-section orthogonal to the puncture direction forms an elliptical shape. The flange part 23b is a disk shaped member formed at the very tip side of the puncture body 23, and the puncture needle 21 protrudes from the center part of the disk. The groove 23c is an indentation formed so as to be sandwiched between the tapered part 23a and the flange part 23b. After use, the puncture body 23 is moved relative to the casing 22 towards the back end side, and a convex portion 22b on the casing 22 is mated with this groove 23c. The puncture body 23 is held inside the casing 22. The insertion part 23d is inserted into a lancet holder 32 of the main body 30, which will be described later. At this time, the tip part of the lancet holder 32 is elastically deformed, and a convex portion 32a formed in the very tip part is mated to the groove 23e. Thus, the puncture body 23 along with the lancet holder 32 is moved forward in the puncture direction and is retracted by the elastic force of the coil spring 31a disposed at the back end side of the lancet holder 32 in the main body 30.
Furthermore, as is shown in Fig. 4, a cap 24 is attached to the puncture needle 21 so as to cover the tip part and the point of the puncture needle 21 is not exposed before use. The cap 24 is formed as a unit with the puncture needle 21 as is the puncture body 23, and part of it is connected to the flange part 23b of the puncture body 23. Therefore, the part where the cap 24 and the flange part 23b are connected is separated by twisting and removing the cap 24 at the time of use, and the puncture needle 21 may be exposed inside the casing 22. In addition, the cap 24 has a protruding part 24a, a cover part 24b and a hole 24c. The protruding part 24a is a part that protrudes in a direction intersecting the puncture direction, and in a state where the cap 24 is attached to the casing 22, it is mated to a groove 22c formed in the end part of the tip side of the casing 22. Thus, the puncture body 23 may be held inside the casing 22 before use. The cover part 24b functions as a cover that covers the very tip part of the casing 22. The hole 24c is a hole formed in close proximity to the very tip of the puncture needle 21 when the puncture needle 21, puncture body 23 and cap 24 are formed as a unit, and the puncture needle 21 is inserted until the cap 24 is separated from the puncture body 23.
The casing 22 is a substantially cylindrical member, and it houses the puncture body 23 inside from before use to disposal after use. In addition, as is shown in Fig. 4, the casing 22 has an inner peripheral surface 22a, the convex portion 22b and the groove 22c. The inner peripheral surface 22a is formed with a slightly larger radius than the radii of the tapered part 23a, the flange part 23b and the like of the puncture body 23, and the puncture body 23 moves to the tip side and the back end side in the puncture direction during use. The convex portion 22b is a member that protrudes to the inside from the inner peripheral surface 22a of the casing 22 and is formed in the vicinity of the middle of the length direction of the casing 22. When the lancet 20 is disposed after use, the puncture body 23 is retracted to the back end side, and the groove 23c mates with the convex portion 22b. Thus, protrusion of the puncture needle 21 from the tip of the casing 22 after use may be avoided, and safety may be assured after use. The groove 22c is an indentation formed in the inner peripheral surface 22a on the tip side of the casing 22. By having the protruding part 24a of the cap 24 mated to the groove 22c before use, the puncture body 23 is held inside the casing 22 so that it does not move to the tip side or the back end side in the puncture direction.

### <Constitution of main body 30>

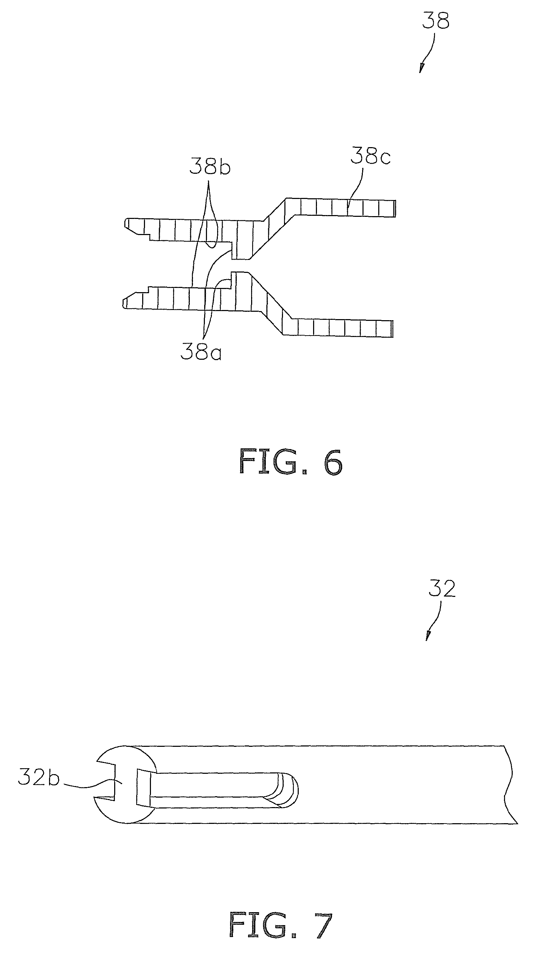

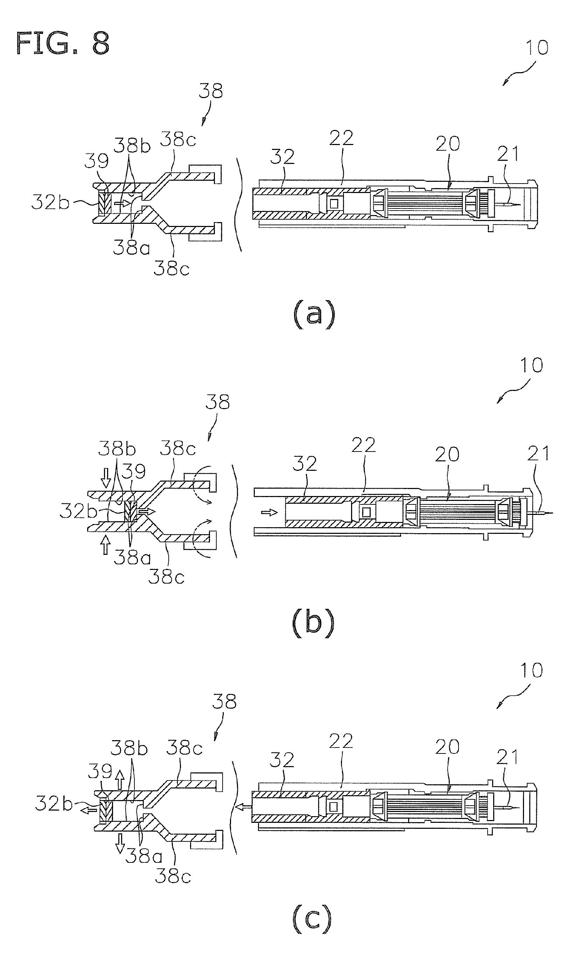

As is shown in Fig. 5, the main body 30 comprises the coil spring 31a, the return spring 31b, the lancet holder 32, a rotating body 33, and a biasing part 34, the housing 35, a disengaging part 36, a setting release button 37 and an abutting stopping member 38, and the lancet 20 is attached as described previously from the tip side thereof (see Fig. 2).
The coil spring 31a is a member that applies force for moving the puncture body 23 of the lancet 20 forward in the puncture direction and is disposed at the back end side of the lancet holder 32. Therefore, when the biasing part 34 is pulled up to a cocked state, the coil spring 31a is in a compressed state and stores the projection energy for the puncture needle 21.
The return spring 31b is a member that applies force for the return to the casing 22 once again after the puncture body 23 of the lancet 20 is projected from the tip of the casing 22 by the coil spring 31 a and is disposed on the inside of the coil spring 31 a. Specifically, when the puncture needle 21 is projected to the tip side by the coil spring 31a described above, the return spring 31b moves from a static state where no load is applied to a compressed state where the return spring 31b is supported at the position in contact with the biasing part 34 and is compressed because of the lancet holder 32 moving to the tip side. Therefore, the force (spring force) that tries to return to the original static state from a compressed state works in the return spring 31b and the puncture needle 21 along with the lancet holder 32 is returned to the back end side. In addition, a spring with a smaller elastic force than the coil spring 31a is used for the return spring 31b. Thus, even when the coil spring 31a and the return spring 31b apply force (spring force) in opposite directions to each other, the force of the coil spring 31a during projection is not hindered and the puncture is performed smoothly.
Moreover, the back-and-forth movement of the lancet holder 32 (puncture needle 21) in the puncture direction because of these two springs 31a and 31b will be described in detail at a later stage.
The lancet holder 32 holds parts (insertion part 23d and groove 23e) of the back end side of the lancet 20 which is inserted from a puncture opening 35a formed in the tip of the housing 35. In addition, the lancet holder 32 has the convex portion 32a mated to the groove 23e on the puncture body 23 on the end part of the tip side. The convex portion 32a is a part that projects to the inside of the cylindrical shaped holder part, and when the insertion part 23d of the puncture body 23 is inserted, the area around convex portion 32a is elastically deformed. Thus, the convex portion 32a may be mated to the groove 23e formed in the back end side of the puncture body 23. Furthermore, as is shown in Fig. 7, the lancet holder 32 has a colliding part 32b in contact with part of the abutting stopping member 38 that will be described later on the end part of the back end side. The colliding part 32b has a surface facing the tip, perpendicular to the puncture direction, to which an elastic part 39, which will be described later, is attached. Furthermore, when the puncture needle 21 is projected, the movement of the puncture needle 21 toward the tip side is controlled by this surface coming into contact with part of the abutting stopping member 38. Moreover, since Fig. 5 is a cross-sectional view, the colliding part 32b is shown in a position separate from the main body of the lancet holder 32, but in actual fact, as is shown in Fig. 7, the main body of the lancet holder 32 and the colliding part 32b are connected in a U shape.
The rotating body 33 is rotated along the periphery centered on the axial direction by rotating a dial part exposed to the outside. In addition the rotating body 33 has a spiral shaped rib 33a that is formed on the outer peripheral surface of the cylindrical part at the tip side of the dial part. This rib 33a mates with a spiral indented part 38d formed on the inner peripheral surface of the abutting stopping member 38, and when the rotating body 33 is moved using a rotary motion, the abutting stopping member 38 with the rotating body 33 may be moved back and forth in the puncture direction. The colliding part 32b (see Fig. 5) formed in the end part of the back end side of the lancet holder 32 is in contact with a contact receiving part 38a on the abutting stopping member 38 when the lancet holder 32 has force applied toward the tip side by the elastic force of the coil spring 31a, and the amount of movement in the puncture direction is controlled (see Fig. 8 (b)). Therefore, the contact position for the colliding part 32b and the collision receiving part 38a may be adjusted in the puncture direction by rotating the rotating body 33. Thus, by rotating the rotating body 33 before performing the puncture of the skin, the amount the puncture needle 21 protrudes at the time of the puncture may be adjusted, and the puncture depth may be controlled.
The biasing part 34 is a member for compressing the coil spring 31a and storing projection energy to project the puncture needle 21 when the puncture body 23 is projected again after the puncture needle 21 attached to the lancet 20 has been projected in the puncture direction and is exposed from the side of the housing 35. The puncture needle 21 may be put into a state where projection is possible by pulling this biasing part 34 toward the back end side, moving the lancet holder 32 toward the back end side, mating a notched part 32c of the lancet holder 32 and a notched part 35c in the case 35 and cocking it.
The housing 35 has the coil spring 31a, the return spring 31b, the lancet holder 32 and the like described above built-in, and constitutes the outer shape of the lancet device 10. In addition, the housing 35, as is shown in Fig. 3 has the puncture opening 35a in the end part of the tip side and an opening 35b where the rotating body 33 is housed in the end part of the back end side. The puncture opening 35a allows the tip of the puncture needle 21 to come out of the opening in the casing 22 when the puncture is performed as well as the insertion of the lancet 20. The opening 35b is formed in a circular shape matching the shape of the rotating body 33.
The disengaging part 36 is exposed on the surface opposite to the surface where the biasing part 34 is exposed on the housing 35, which is in a substantially rectangular shape and is disposed inside of the housing 35 so as to be in contact with the end part of the back end side of the casing 22. After completion of the puncture, the disengaging part 36 is moved to the tip side, and since, first of all, only the casing 22 moves forward towards the tip side, a convex portion 22b in the casing 22 mates with the groove 23c of the puncture body 23. Furthermore, when the disengaging part 36 moves further forward, the hold on the puncture body 23 (insertion part 23d and groove 23e) is released by part of the disengaging part 36 pushing up part of the protruding part of the lancet holder 32, and the lancet 20 is taken out of main body 30.
The setting release button 37 is a member for releasing the set state where the puncture needle 21 can be projected when it has been cocked with the lancet 20 attached or after it has been projected with the lancet 20 attached and it has been cocked again by the biasing part 34 and is exposed on the outside of the housing 35. Therefore, after the puncture needle 21 is set to a projectable state when the puncture is performed, the puncture needle 21 is projected in the puncture direction by pressing this setting release button 37 and releasing the cocking of the notch part 32c of the lancet holder 32 and the notch part 35c of the case 35 as described above.
As is shown in Fig. 5, the abutting stopping member 38 is inside the housing 35 and is disposed in the vicinity of the rotating member 33. Furthermore, the abutting stopping member 38 is connected to the rotating body 33, and moves back and forth in the puncture direction along with the movement of the rotating body 33. In addition, as is shown in Fig. 6, the abutting stopping member 38 has the collision receiving part 38a, contact parts (first contact part and a second contact part) 38b and a base 38c. The collision receiving part 38a has a collision receiving surface that collides a vertical surface of the colliding part 32b with respect to the puncture direction formed on the back end side of the lancet holder 32 described above and. As with the surface of the colliding part 32b on the lancet holder 32 side, this collision receiving surface is a vertical surface in the puncture direction and is divided into two parts. The contact parts 38b have two surfaces substantially parallel in the puncture direction disposed so as to be opposed to each other, and because the abutting stopping member 38 as a whole elastically deforms with the collision between the colliding part 32b and the collision receiving part 38a, there is contact with side surface of the colliding part 32b. The movement of the colliding part 32b is controlled. When the colliding part 32b and the collision receiving part 38a collide, the base 38c elastically deforms toward the inner peripheral part (see the dotted and dashed lines in Fig. 8 (b)).
An elastic part 39 is attached to the surface of the colliding part 32b, which is a part of the lancet holder 32, on the side of colliding with the collision receiving part 38a and is resin foam having elasticity in the puncture direction. When the colliding part 32b and the collision receiving part 38a collide, this elastic part 39 functions as a cushion, and assures that there may be a long deformation time for the abutting stopping member 38 because of the collision.

### <Description of the lancet device 10 operation>

As is shown in Fig. 2, when the use of the lancet device 10 of the present embodiment begins, an unused, new lancet 20 is first inserted into the puncture opening 35a of the main body 30. When the lancet 20 is inserted deep into the puncture opening 35a, the insertion part 23d formed on the end part of the back end side of the puncture body 23 passes through part of the convex portion 32a of the lancet holder 32 and is inserted deep inside. Furthermore, at the same time as the attachment of the lancet 20 to the main body 30 is completed by making the groove 23e formed so as to be adjacent to the insertion part 23d mate with the convex portion 32a of the lancet holder 32, it is cocked and put into the projection ready state for the puncture needle 21.
Next, the cap 24 that is formed as a unit with the puncture body 23 is removed to expose the puncture needle 21. Since part of the cap 24 is in contact with the surface on the tip side of the flange part 23b of the puncture body 23, the cap 24 is rotated and removed, with this connected part twisted off.
Next, with the puncture opening 35a in a state of contact with the skin to be punctured, the tip part of the puncture needle 21 protrudes a prescribed amount from the puncture opening 35a formed in the very tip side of the main body 30 when the setting release button 37 is pressed. Furthermore, the puncture needle 21 returns once again into the casing 22 because of the force (spring force) applied by the return spring 31b after the puncture.
Here, when the puncture needle 21 returns to the inside of the casing 22 because of the return spring 31b after the puncture, the coil spring 31a for projection may be compressed once again by that force. In this case, the puncture needle 21 once again moves towards the tip side along with the lancet holder 32 because of the energy stored when the coil spring 31a is compressed. Therefore, there is a risk of an unnecessary puncture being performed after the puncture, a new puncture wound formed in the user and there being unnecessary suffering. Furthermore, this back-and-forth movement of the puncture needle 21 in the puncture direction is repeated between the two springs, and two or more punctures may occur.
Conventional lancet devices have the lancet holder come into contact with a brake member, and apply a braking force using friction to the lancet holder when it moves forward for this problem, and prevent multiple punctures. However, with this constitution, the size of the braking force for the lancet holder depends on the dimensional precision of the parts for the lancet holder and the brake member. Therefore, to obtain a stable braking force, it is necessary to manage the forming of the parts with a high dimensional precision.
When the colliding part 32b, which is a part of the very back end part of the lancet holder 32, and the collision receiving part 38a which is a part of the abutting stopping member 38, collide in the lancet device 10 of the present embodiment, the force of the back-and-forth motion of the lancet holder 32 in the puncture direction is weakened afterwards by the abutting stopping member 38 being elastically deformed. More specifically, when the lancet holder 32 is moved forward (see Fig. 8 (a)) by the force applied by the coil spring 31a, the colliding part 32b, which is a part of the lancet holder 32 collides with the collision receiving part 38a, which is a part of the abutting stopping member 38. At this time, the abutting stopping member 38 as a whole is elastically deformed toward the inside (direction intersecting the puncture direction) as is shown in Fig. 8 (b) because of the collision between the colliding part 32b and the collision receiving part 38a. For example, the collision receiving part 38a is partially deformed so as to move toward the tip side, and the contact part 38b is deformed so as to move to the inner peripheral side of the housing 35, that is in the direction of contact with the side surface of the colliding part 32b, which is a part of the lancet holder 32. Furthermore, the base 38c, which is in contact with another member inside the housing 35 is elastically deformed so as to bend toward the inner peripheral side as a fulcrum on the very tip side as shown in Fig. 8 (b).
Thus, when the colliding part 32b and the collision receiving part 38a collide, the contact part 38b comes into contact with the side surface of the colliding part 32b, and is in a state where it is momentarily held, and braking may be applied to the colliding part 32b, that is on the retraction of the lancet holder 32 and the force weakened.
In the lancet device 10 of the present embodiment, the collision energy when the colliding part 32b and the collision receiving part 38a collide may be converted into energy (braking force) for weakening the force when the colliding part 32b retracts because of the force applied to the lancet holder 32 by the return spring 31b. Therefore, the force on the lancet holder 32 that returns into the casing 22 because of the return spring 31b is weakened greatly by the braking force when the contact part 38b comes into contact with the side surface of the colliding part 32b, so the amount of compression of the coil spring 31a may be made smaller than the conventional after the puncture. Therefore, the energy stored with the compression of the coil spring 31a is reduced, and the amount that the puncture needle is driven forward toward the tip side once again may be reduced. Therefore, after the puncture, the amplitude of the back-and-forth motion of the puncture needle 21 in the puncture direction is greatly reduced over the conventional, and occurrences of multiple punctures may be avoided.
In addition, when the collision energy is large in a constitution such as that of the present embodiment, that is when the force returning the puncture needle 21 into the casing 22 is large, and the risk of multiple punctures is high, the elastic deformation energy for the abutting stopping member 38 is large during the collision. Therefore, when the collision energy is large, the elastic deformation of the abutting stopping member 38 is large, and the part (colliding part 32b) of the lancet holder 32 may be made to contact the contact part 38b for a long time with a stronger intensity. As a result, the braking force required for avoiding multiple punctures may be obtained according to the size of the energy during the collision, and the necessity for managing the dimensions of the lancet holder 32 and the abutting stopping member 38 with high precision is reduced.
Furthermore, an elastic part 39 having elasticity in the puncture direction is attached to the colliding surface side of the colliding part 32b. Therefore, the contact time for the colliding part 32b and the collision receiving part 38a during the collision is increased, and a long time for braking that is applied by the part (colliding part 32b) of the lancet holder 32 and the part (contact part 38b) of the abutting stopping member 38 may be assured.
Moreover, after the puncture is completed, the lancet 20 is removed from the main body 30 by the disengaging part 36 and disposed of. Removal of the lancet 20 from the main body 30 is done using the disengaging part 36. In other words, the hold on the puncture body 23 by the lancet holder 32 is released by moving the disengaging part 36 toward the tip side, and the lancet 20 may be removed from the puncture opening 35a

### <Features of the present lancet device 10>

### (1)

As is shown in Fig. 5, in the lancet device 10 of the present embodiment, the puncture needle 21 is received the biasing force applied by the coil spring 31a and is projected toward the tip side, and is retracted by the biasing force applied by the return spring 31b. As is shown in Fig. 8 (b), in the lancet device 10, the colliding part 32b and the collision receiving part 38a are made to collide when the puncture needle 21 is projected, and the abutting stopping member 38 elastically deforms. The colliding part 32b is a part of the lancet holder 32 that holds the lancet 20 having the puncture needle 21. The collision receiving part 38a is a part of the abutting stopping member 38. Furthermore, the contact part 38b of the abutting stopping member 38 moves in the direction of the colliding part 32b and comes into contact with the colliding part 32b by the elastic deformation of this abutting stopping member 38, and the force that retracts the lancet holder 32 is weakened.
Thus, by using the collision energy of the lancet holder 32 and the abutting stopping member 38, the amplitude and number of back-and-forth movements of the lancet holder 32 in the puncture direction after the puncture may be reduced without managing the dimensional precision of the abutting stopping member 38 and the like at a high level that apply braking to the movement of the lancet holder 32, and multiple punctures by the puncture needle 21 may be prevented stably.
In addition, the larger the collision energy is, the larger the elastic deformation of the abutting stopping member 38 is. So the contact force between the contact part 38b and the colliding part 32b is greater and the contact time is longer, and multiple punctures may be avoided more effectively.

### (2)

In the lancet device 10 of the present embodiment there are the contact parts 38b that are part of the abutting stopping member 38 and include surfaces that are substantially parallel to the puncture direction and opposed to each other, and the colliding part 32b formed in the very back end part of the lancet holder 32 passes between these contact parts 38b and collides with the collision receiving part 38a, which is part of the abutting stopping member 38.
Thus, the contact part 38b comes into contact with the side surface of the colliding part 32b because of the elastic deformation of the abutting stopping member 38 and may weaken the force retracting the lancet holder 32. As a result, even though it is a simple constitution, the abutting stopping member 38 is deformed by the collision of the colliding part 32b and the collision receiving part 38a, and occurrences of multiple punctures may be effectively prevented.

### (3)

The lancet device 10 of the present embodiment is provided with an elastic part 39 on the surface of the colliding side with the collision receiving part 38a in the colliding part 32b of the lancet holder 32.
Thus, when the colliding part 32b collides with the collision receiving part 38a, the elastic part 39 operates as a buffering material, and the collision time between the colliding part 32b and the collision receiving part 38a may be increased. Therefore, the time that the abutting stopping member elastically deforms is increased, and the movement of the part of the lancet holder 32 (colliding part 32b) may be more effectively limited. As a result, multiple punctures by the puncture needle 21 may be avoided by the reduction of the amplitude of the back-and-forth movement of the lancet holder 32 in the puncture direction after the puncture.

### (4)

In the lancet holder 10 of the present embodiment, two springs are used, a coil spring 31a for the first biasing part for projecting the puncture needle 21 toward the tip side, and a return spring 31b for the second biasing part that retracts the puncture needle 21 along with lancet holder 32 after the puncture.
Thus, even when there is back-and-forth movement of the puncture needle 21 along with the lancet holder 32 between the two springs, multiple punctures may be effectively avoided by a part of abutting stopping member 38 coming into contact with a part of the lancet holder 32 and the retracting force being weakened by the elastic deformation of the abutting stopping member 38 at the time of the collision.

### <Embodiment 2>

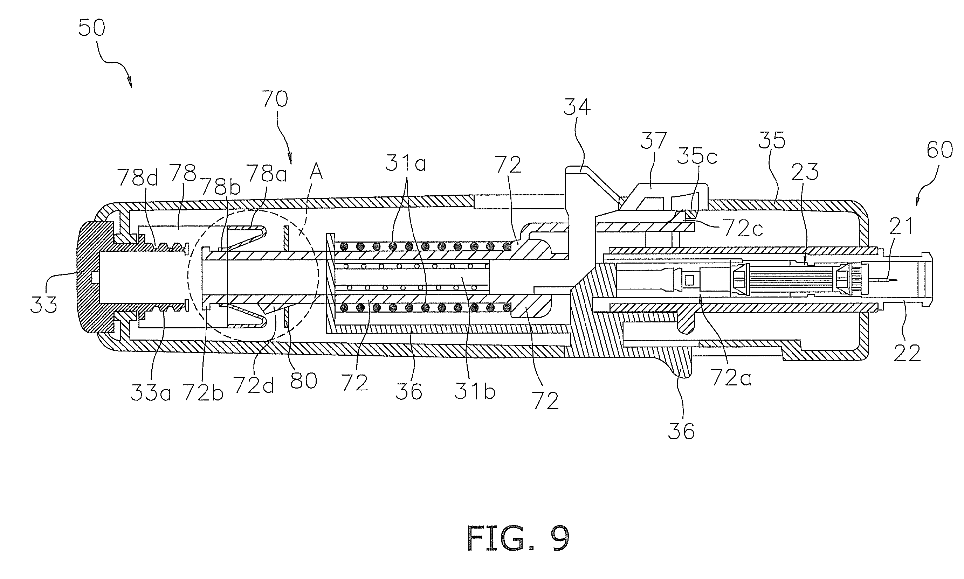

The lancet device according to an another embodiment of the present invention will be described as follows using Fig. 9 through Fig. 11 (c).
Moreover, of the various parts included in the lancet device (puncture device) 50 according to the present embodiment, the parts that were described in Embodiment 1 above and have the same functions are given the same element numbers and descriptions will be omitted as a matter of convenience for the description. In addition as in Embodiment 1 described above, the "tip side" used in the description hereinafter is the side where the tip of the lancet 20 puncture needle 21, which will be described below, protrudes and the "back end side" is the side opposite to this.

### <Constitution of the lancet device 50>

The lancet device 50 of the present embodiment is provided with a lancet 60 and a main body 70 as shown in Fig. 9.
The lancet 60 has the puncture needle 21 for forming the puncture wound inside, and it is attached from the tip side of the main body 70.
The main body 70 incorporates the coil spring (first biasing part) 31a and the return spring (second biasing part) 31b. The coil spring 31a applies force to the puncture needle 21 for making it protrude in a prescribed puncture direction. The return spring 31b returns the puncture needle 21 that has been projected by the coil spring 31a into the housing 35.

### <Constitution of the lancet 60>

The lancet 60, as is shown in Fig. 9, is provided with a substantially cylindrical casing 22 and a puncture body 23 housed in a movable state to the tip side and the back end side in the puncture direction in the casing 22 when the lancet device 10 is used.

### <Constitution of main body 70>

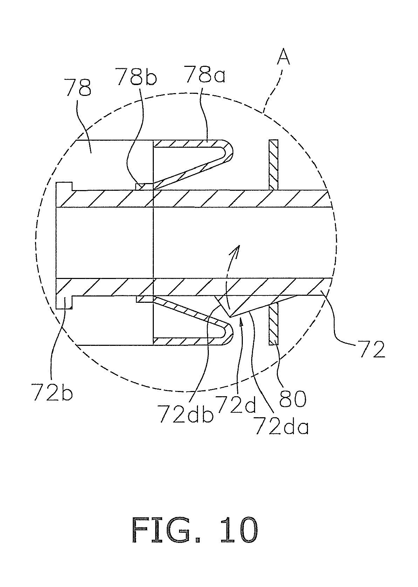

As is shown in Fig. 9, the main body 70 comprises the coil spring 31a, the return spring 31b, the lancet holder 72, the rotating body 33, and the biasing part 34, the housing 35, the disengaging part 36, the setting release button 37 and the abutting stopping member 78. The lancet 60 is attached as described previously from the tip side thereof.
A rib member (pressing pressure part) 80 is formed in a position offset to the back end side of the lancet holder 72 inside the housing 35. The rib member 80 extends substantially perpendicular to the puncture direction. This rib member 80 applies pressing pressure to an elastic member 72d, which is a part of the lancet holder 72 described later, in a direction intersecting the puncture direction when the lancet holder 72 moves in the puncture direction and has a function for applying braking to the movement of the lancet holder 72.
The lancet holder 72 holds parts of the back end side of the lancet 60 which is inserted into the puncture opening 35a formed in the tip of the housing 35. Furthermore, as is shown in Fig. 10, the lancet holder 72 has a colliding part 72b in contact with part of an abutting stopping member 78 described later on the end part of the back end side. The colliding part 72b has a surface substantially perpendicular to the puncture direction. Furthermore, when the puncture needle 21 is projected, the movement of the puncture needle 21 and the lancet holder 72 toward the tip side is regulated by the surface coming into contact with the collision receiving part 78b, which is a part of the abutting stopping member 78. Furthermore, the lancet holder 72 has an elastic member 72d that deforms elastically in a direction intersecting the puncture direction in a position offset to the back end side.
The elastic member 72d comes into contact with the end part of the rib member 80 formed inside the housing 35 when the lancet holder 72 moves back and forth in the puncture direction and elastically deforms in a direction that intersects the puncture direction centered on the center of rotation of the end part on the tip side connected to the body part of the lancet holder 72 in the puncture direction. In addition, the elastic member 72d has a first sloped surface 72da that is sloped toward the tip side with respect to the puncture direction and a second sloped surface 72db sloped toward the back end side with a slope angle that is greater than the first sloped surface 72da with respect to the puncture direction. Thus, by the different slope angles of the contact surfaces (first sloped surface 72da and second sloped surface 72db) which is in contact with the rib member 80, of the elastic member 72d which deforms elastically, a strong braking force may be applied when the lancet holder 72 moves toward the back end side but a week braking force may be applied when the lancet holder 72 moves toward the tip side. More specifically, when the first sloped surface 72da, which has a comparatively small slope angle, and the rib member 80 come into contact, that is when the lancet holder 72 moves toward the tip side in the puncture direction, it may cause elastic deformation of the elastic member 72d toward the inside intersecting the puncture direction by a comparatively small force because the slope is gentle. On the other hand, when the second sloped surface 72db, with its slope angle that is larger than the first sloped surface 72da, come into contact with the rib member 80, that is when the lancet holder 72 moves toward the back end side, a greater force is required to cause the elastic member 72d to elastically deform toward the inside intersecting the puncture direction than when the elastic member 72d is elastically deformed while there is contact with the first sloped surface 72da since the slope is greater than the first sloped surface 72db. Therefore, when the lancet holder 72 is moved toward the tip side in the puncture direction, a small braking force may be applied to the lancet holder 72 because the gently sloped first sloped surface 72da and the rib member 80 come into contact. On the other hand, when the lancet holder 72 is moved toward the back end side in the puncture direction, a large braking force may be applied to the lancet holder 72 because the severely sloped second sloped surface 72db and the rib member 80 come into contact.
Moreover, the application of the braking force on the lancet holder 72 because of the contact between the elastic member 72d of the lancet holder 72 and the rib member 80 will be described in detail at a later stage.
As is shown in Fig. 9 and Fig. 10, the abutting stopping member 78 is inside the housing 35 and attached to the rotating body 33. With movement of the rotating body 33, it moves back and forth in the puncture direction. Thus, the amount that the puncture needle 21 tip protrudes may be adjusted. In addition, the abutting stopping member 78 has a protruding part 78a and a collision receiving part 78b. The protruding part 78a is a member that protrudes toward the tip side in the puncture direction, and the collision receiving part 78b is on the end part thereof. This collision receiving part 78b has a surface that is substantially vertical in the puncture direction as with the surface of the colliding part 72b on the lancet holder 72 side, and the movement of the lancet holder 72 forward in the puncture direction is controlled with this surface.

### <Description of the lancet device 50 operation after projection>

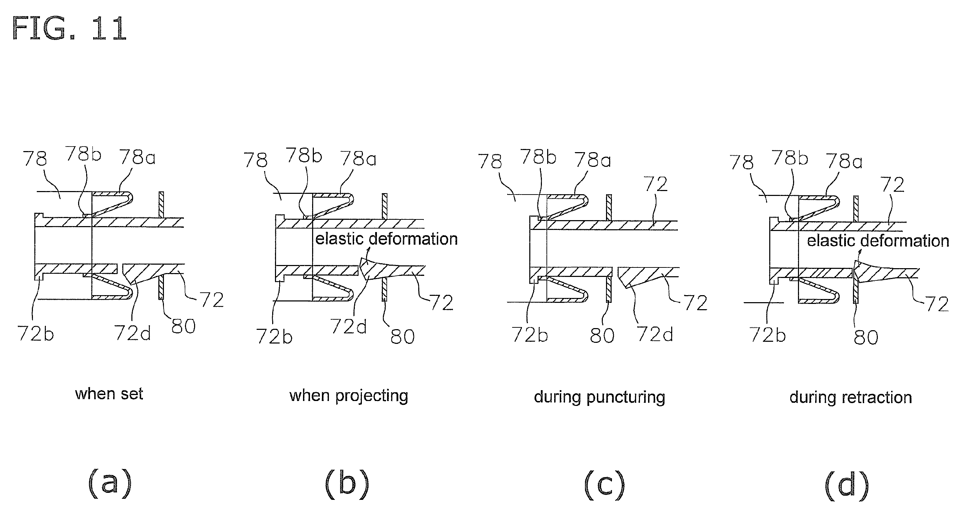

When the lancet 60 is set in the main body 70 in the lancet device 50 of the present embodiment, the rib member 80 formed in the back end side inside the main body as shown in Fig. 11 (a) and the elastic member 72d formed on the back end side of the lancet holder 72 are in a state where they are not in contact with each other.
Furthermore, when the setting release button 37 is pressed and the lancet holder 72 is projected toward the tip side, the rib member 80 and the first sloped surface 72da of the elastic member 72d come into contact as is shown in Fig. 11 (b), and the lancet holder 72 moves forward in the puncture direction while in elastically deforming the elastic member 72d toward the inside intersecting the puncture direction. Furthermore, as is shown in Fig. 11 (c), the puncture is performed by the puncture needle 21 in a position where the rib member 80 has crossed over the first sloped surface 72da of the elastic member 72d.
Here, after the puncture, the lancet holder 72 moves back and forth in a prescribed range while repeatedly receiving the elastic force from the two springs (coil spring 31a for projection and return spring 31b) in the same manner as the lancet holder 32 in Embodiment 1.
However, when the lancet holder 72 returns to the vicinity of the position where it was set as shown in Fig. 11 (a) after the puncture, the second sloped surface 72db of the elastic member 72d comes into contact with the rib member 80 as shown in Fig. 11 (d), and the elastic member 72d is once again in elastically deformed toward the inside intersecting the puncture direction. At this time, the second sloped surface 72db of the elastic member 72d that is in contact with the rib member 80 has a greater slope with respect to the puncture direction than the first sloped surface 72da as mentioned above, so a braking force that is larger than the braking force applied to the lancet holder 72 when the projection shown in Fig. 11 (b) is applied to the lancet holder 72.
Subsequently, the rib member 80 crosses over the second sloped surface 72db of the elastic member 72d shown in Fig. 11 (d) and moves to a state where the first sloped surface 72da and the rib member 80 are in contact as shown in Fig. 11 (b). Next, the root part of the sloped surface of the first sloped surface 72d and the rib member 80 come into a state of contact as shown in Fig. 11 (a). After this, furthermore, the lancet holder 72 moves toward the tip side because of the coil spring 31a for projection has been compressed once again, but as is shown in Figure. 11 (b), this time the first sloped surface 72da and the rib member 80 come into contact and a braking force is applied to the lancet holder 72.
While the lancet holder 72 moves back and forth as above after the puncture in the lancet device 50, the lancet holder 72 back-and-forth movement is effectively dampened by applying a braking force as described above to the movement toward both directions, the tip side and the back end side, and a second puncture may be prevented after the puncture.
The site of the damping mechanism (elastic member 72d and rib member 80) for damping the back-and-forth movement of the lancet holder 72 after the puncture functions is provided in the vicinity of the lancet holder 72 position at the time it is set as shown in Fig. 11 (a) as mentioned above in the lancet device 50 of the present embodiment.
Thus, the lancet holder 72 that has been returned toward the back end side by the elastic force from the return spring 31b once again compresses the coil spring 31a for projection because of the return force, and the force of the lancet holder 72 movement toward the tip side from the elastic force of the coil spring 31a may be weakened. In other words, when the lancet holder 72 is returned by the return spring 31b, the elastic energy stored in the coil spring 31a for projection may be reduced by the application of braking force to the lancet holder 72 because of the contact of the elastic member 72d and the rib member 80. Thus, the braking force is applied to the lancet holder 72 that is moving back and forth in the puncture direction after the puncture, and the back-and-forth movement may be dampened.
In addition, in the present embodiment, the application of the braking force to the lancet holder 72 by making this rib member 80 come into contact with the elastic member 72d is carried out at the back end side of the lancet device 50. Thus, even if foreign matter and the like enters in the tip side of the lancet device 50 from the opening part, entry of this foreign matter and the like into the mechanism that applies the braking force giving rise to problems in operation may be avoided.
Furthermore, the first sloped surface 72da and the second sloped surface 72db of the elastic member 72d have different slope angles in the puncture direction, so a difference in the size of the braking force applied to the lancet holder 72 may be provided between when the lancet holder 72 moves toward the tip part and when the lancet holder 72 moves toward the back end part in the puncture direction. Thus, by making the braking force during movement toward the tip side smaller, the puncture may be performed smoothly with hardly any braking being applied at the first sloped surface 72da, which has a gentle slope, during the puncture. Furthermore, the so-called second stab after the puncture may be prevented by applying a large braking force using the second sloped surface 72db, which has a slope that is larger than the first sloped surface 72da, to the lancet holder 72 when it is retracted after the puncture and damping the back-and-forth movement of the lancet holder 72.

### <Features of the present lancet device 50>

### (1)

When the lancet 60 is set in the lancet holder 72 in the lancet device 50 of the present embodiment, the elastic member 72d of the lancet holder 72 and the rib member 80 are in a state of noncontact as shown in Fig. 11 (a). Furthermore, when the lancet holder 72 moves to the vicinity of the set position shown in Figs. 11 (a) after the puncture, the rib member 80 and the elastic member 72d come into contact and the lancet holder 72 moves while it elastically deforming the elastic member 72d.
Thus, a braking force may be applied to the lancet holder 72, which is moving back and forth in the puncture direction by the coil spring 31a and the return spring 31b after the puncture, in the vicinity of the set side. As a result, the amount that the lancet holder 72 that returns to the back end side in the puncture direction after the puncture compresses the coil spring 31a for projection may be reduced, and the back-and-forth movement of the lancet holder 72 after the puncture may be effectively dampened.
In addition, since the mechanism (elastic member 72d and rib member 80) that applies the braking force to the lancet holder 72 is provided on the back end side of the lancet device 50, even if foreign matter or the like enters from the tip side where the lancet 60 is attached and detached, occurrences of operating problems in the mechanism described above may be prevented.

### (2)

Of the mechanisms (rib member 80 and elastic member 72d) for applying braking force to the lancet holder 72, which is moving back and forth after the puncture in the lancet device 50 of the present embodiment, sloped surfaces (first sloped surface 72da and second sloped surface 72db) with different sizes of slope angles with respect to the puncture direction are formed on the sides of the elastic member 72d as shown in Figure 10.
Thus, the size of the braking force applied to the lancet holder 72 may be made different because the angles of the sloped surfaces in contact with the rib member 80 during the movement to the tip side and the movement to the back end side in the puncture direction are different.
As a result, the braking force of a suitable size may be applied to each direction, forward and backward in the puncture direction, and the back-and-forth movement of the lancet holder 72 may be effectively prevented.

### (3)

In the lancet device 50 of the present embodiment, the slope angle of the second sloped surface 72db sloping toward the back end side is larger than the first sloped surface 72da sloping towards the tip side in the puncture direction for the two sloped surfaces sloped with respect to the puncture direction and formed on the elastic member 72d.
Thus, a small braking force may be applied to the lancet holder 72 moving toward the tip side in the puncture direction, on the other hand, a braking force larger than that for the tip side may be applied to the lancet holder 72 moving toward the back end side in the puncture direction. As a result, a large braking force may be applied to the lancet holder 72 that has returned after the puncture without hindering the forward movement of the lancet holder 72 toward the tip side at the time of the puncture, and the back-and-forth movement of the lancet holder 72 after the puncture may be effectively dampened. The risk of second stabs may be avoided.

### (4)

In the lancet device 50 of the present embodiment, the rib member 80, which is formed so as to extend in a substantially perpendicular direction to the puncture direction on the back end side of the housing 35, is used as a pressing pressure member that is contact with the elastic member 72d which is a part of the lancet holder 72 and applies the braking force to the lancet holder 72.
Thus, by using a simple constitution and pressing the elastic member 72d, the back-and-forth movement of the lancet holder 72 may be dampened.

### <Other Embodiments>

A description has been given above of embodiments of the present invention, but the present invention is not limited to the embodiments described above, and there may be various changes within a scope that does not deviate from the gist of the invention.

### (A)

In Embodiment 1, the constitution shown in Fig. 5 was cited and described as an example of the colliding part 32b formed as a part of the lancet holder and the collision receiving part 38a and contact part 38b formed as a part of the abutting stopping member 38. However, the present invention is not limited to this.
For example, even if there is a constitution where the shapes, size and direction of deformation of the colliding part 32b, the collision receiving part 38a and the contact part 38b are different, an effect that is the same as above may be obtained if the constitution has an action that deforms in the contact part 38b and weakens the retraction force of the colliding part 32b (lancet holder 32) when the colliding part 32b and the collision receiving part 38a collide.

### (B)

In Embodiment 1, an example provided with the elastic part 39 between the colliding part 32b and the collision receiving part 38a was cited and described. However, the present invention is not limited to this.
For example, there may be a constitution where the elastic part 39 is not provided, and there may be a constitution such that the colliding part 32b and the collision receiving part 38a directly collide.
However, the constitution in Embodiment 1 is preferable because the time of elastic deformation of the abutting stopping member 38 as a whole during the collision is increased, and the time that the colliding part 32b is inserted between the contact parts 38b is increased as in Embodiment 1. As a result, the back-and-forth motion of the lancet holder 32 in the puncture direction may be controlled, and multiple punctures may be more effectively prevented.

### (C)

In Embodiment 1, an example where the elastic part 39 is attached to the colliding surface side of the colliding part 32b, which is a part of the lancet holder 32 was cited and described. However, the present invention is not limited to this.
For example, the elastic part 39 may be provided on the colliding surface side of the collision receiving part 38a, which is a part of the abutting stopping member 38. In this case also, an effect that is the same as the effect described in the embodiment above may be obtained.
Moreover, the material for the elastic part 39 is not limited to the resin foam described in the embodiment above, and an another elastic body, such as rubber or a spring, may be used.

### (D)

In Embodiment 1 described above an example where the abutting stopping member 38 as a whole is elastically deformed when the colliding part 32b and the collision receiving part 38a collide, weakening the retraction force of the colliding part 32b was cited and described. However, the present invention is not limited to this.
For example, there may be a constitution that is not limited to elastic deformation such that the colliding part 32b is weakened the retraction force by deforming the abutting stopping member 38 during the collision described above.
However, in the lancet device 10 that is reused, where the lancet 20 is replaced after the first puncture, it is preferable to be able to have repeated use with elastic deformation as in the embodiment described above.
In addition, the elastic deformation of the abutting stopping member 38 is not limited to being extended to the whole is in the embodiment described above, and the constitution may be one where the retracting force of the lancet holder 32 is weakened by elastic deformation of a part thereof.

### (E)

In Embodiment 1 described above, an example where the lancet device according to the present invention is used in the lancet device 10, which includes the lancet 20 was cited and described. However, the present invention is not limited to this.
For example, the lancet device according to the present invention may be a constitution that does not include the lancet 20, that is a constitution of just the main body 30 in which the lancet 20 is not inserted into the lancet holder 32.

### (F)

In Embodiment 2, an example given a mechanism that dampens the back-and-forth movement of the lancet holder 72 after the puncture and uses the elastic member 72d, which is a part of the lancet holder 72, and the rib member 80, which is formed inside the housing 35, was cited and described. However, the present invention is not limited to this.
For example, the shape, position and the like of the elastic member used as the damping mechanism described above is not limited to the shape and the like described in

### Embodiment 2.

Furthermore, the shape, position and the like of the rib member that comes into contact with the elastic member and applies pressing pressure is not limited to the shape and the like described in Embodiment 2.

### (G)

In Embodiment 2 described above, an example where two surfaces having different slope angles for the elastic member 72d that comes into contact with the rib member 80 are formed as a mechanism that applies braking forces of different sizes backwards and forwards in the puncture direction was cited and described. However, the present invention is not limited to this.
For example, a mechanism that uses contact between other the members themselves may be used as the mechanism for applying braking forces of different sizes between backwards and forwards in the puncture direction.

### (H)

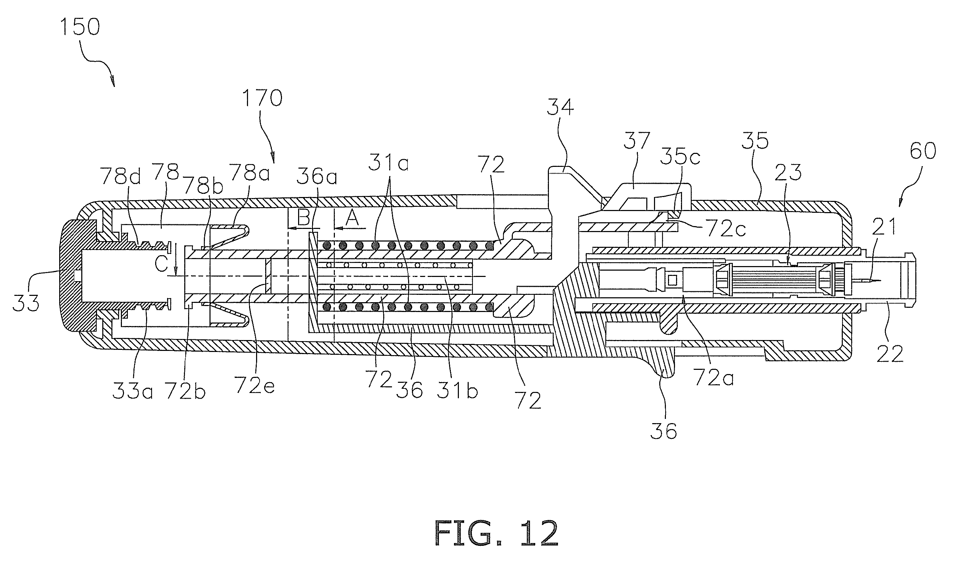

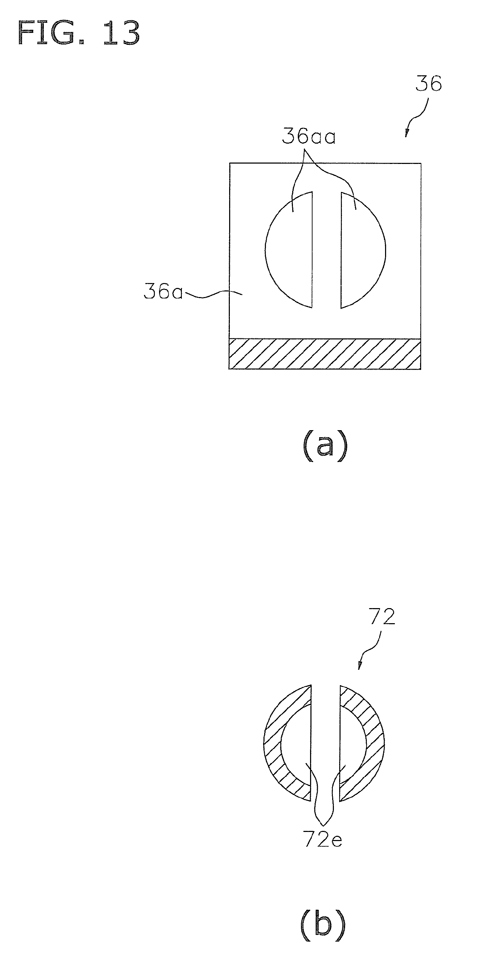

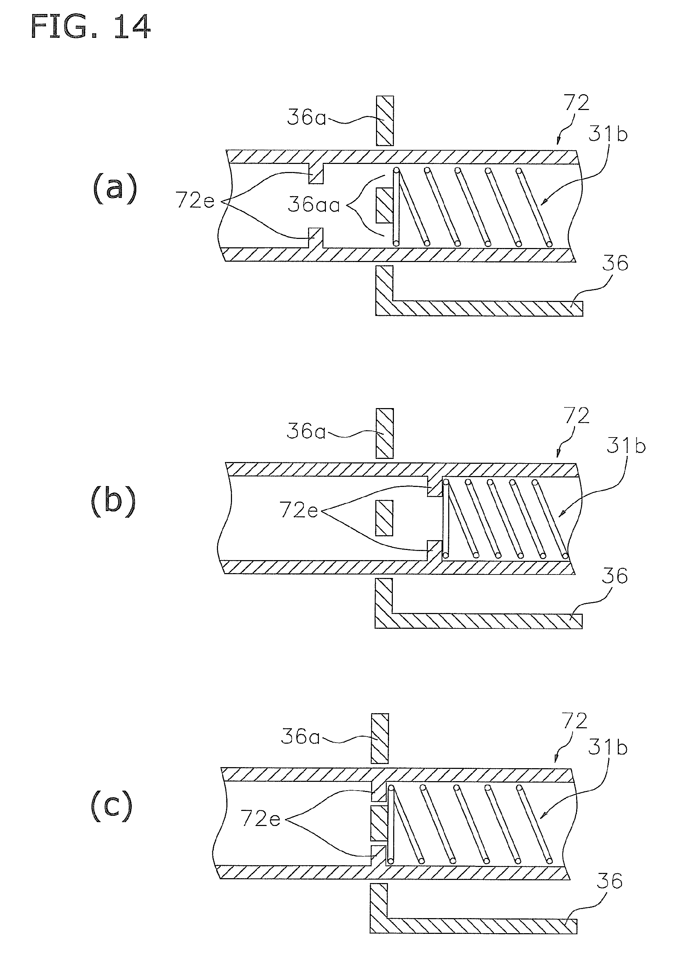

In Embodiment 2, as is shown in Fig. 9 and the like, an example where the braking force is applied to the lancet holder 72 that is moving back and forth in the puncture direction in the vicinity of the setting side and the back-and-forth movement of the lancet holder 72 by the coil spring 31a and the return spring 31b after the puncture is effectively dampened by the rib member 80 and the elastic member 72d coming into contact and the lancet holder moving as it elastically deforms the elastic member 72d was cited and described. However, the present invention is not limited to this.
As is shown in Fig. 12 for example, there may also be a lancet device 150 that includes a main body 170 provided with a rib (contact part) 72e on the inside surface somewhat to the rear of the middle along the length of the lancet holder 72 and an opening aa in a wall part 36a the of the disengaging part 36 through which the lancet holder 72 passes.
The lancet device 150 passes the back end part of the lancet holder 72, which has a cross-section where two substantially half rounds are disposed in opposition with a prescribed gap as shown in Fig. 13 (b) through the opening 36aa formed in the wall part 36a, which includes the disengaging part 36 and is arranged so as to stand substantially perpendicular to the puncture direction as shown in Figs. 13 (a).
At this time, the rib 72e shown in Figs. 13 (b) protrudes toward the inside from both sides in a direction that intersects the puncture direction in a plan view, as is shown in Fig. 14 (a) through Fig. 14 (c). Therefore, moving from the state before puncture (see Fig. 14 (a)) to that during puncture, there is forward movement while pressing the back end part of the return spring 31b in the puncture direction after passing through the opening 36aa in the wall part 36a of the disengaging part 36. Since elastic energy is stored in the return spring 31b here, the rib 72e returns to the position of the wall part 36a and stops after the puncture as is shown in Fig. 14 (c).
Thus, the movement of the lancet holder after the puncture may be controlled, and the risk of a second stab may be avoided.

### (I)

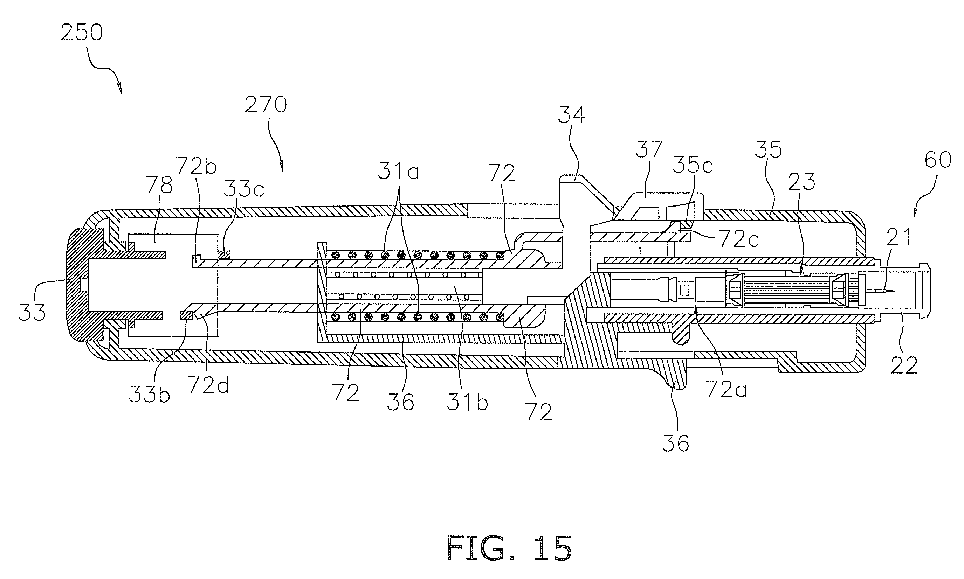

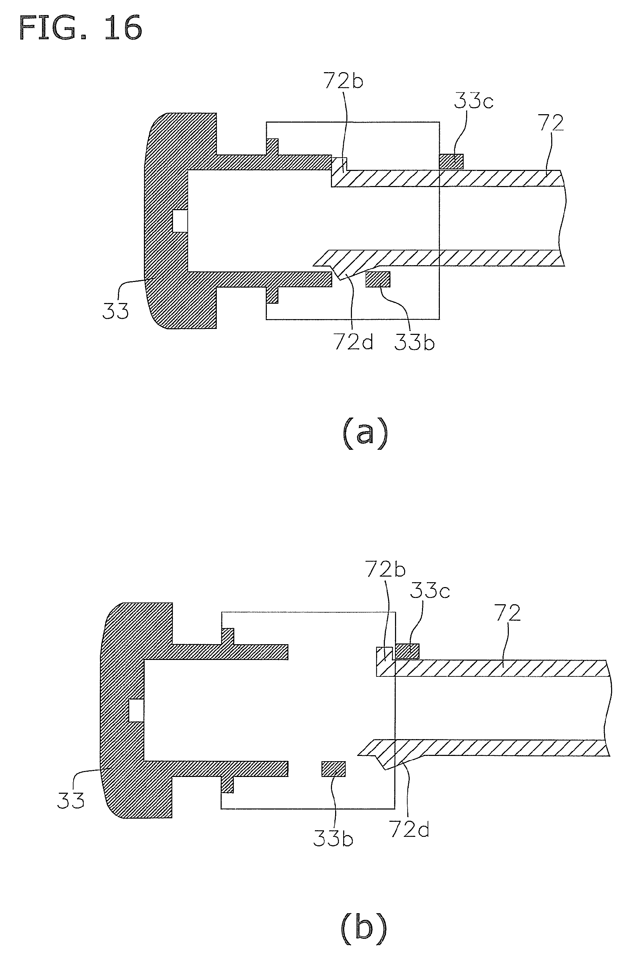

In Embodiment 2, as is shown in Fig. 9 and the like, an example where the braking force is applied to the lancet holder 72 that is moving back and forth in the puncture direction in the vicinity of the setting side and the back-and-forth movement of the lancet holder 72 by the coil spring 31a and the return spring 31 b after the puncture is effectively dampened by the rib member 80, which is formed on the inside surface toward the back end part in the housing 35, and the elastic member 72d coming into contact and the lancet holder moving as it elastically deforms elastic member 72d was cited and described. However, the present invention is not limited to this.
For example, there may be a lancet device 250 that, instead of the rib member 80, is provided with an abutting stopping part 33c made in a stepwise shape such that the abutting stopping surface goes forward and backward in the puncture direction by rotating a second stab prevention rib (pressing pressure part) 33b as a part of the rotating body (puncture depth adjusting member) 33 and the rotating body (puncture depth adjusting member) 33 as shown in Fig. 15 and includes a main body 270 provided with a constitution that controls the movement of the elastic member 72d, which has a protrusion formed in the back end part of the lancet holder 72 and the colliding part 72b.
In this case also, the elastic member 72d moves forward with the protruding member in contact with the rib 33b as is shown in Fig. 16 (a) before the puncture. Furthermore, after the puncture, and after the abutting stopping part 33c and the colliding part 72b come into contact as is shown in Fig. 16 (b), there is backward movement by the elastic force of the return spring 31b, but the protruding part of the elastic member 72d moves backward again while in contact with the rib 33b.
Thus, the amount that the lancet holder 72 that returns to the back end side in the puncture direction after the puncture compresses the coil spring 31a for projection may be reduced, and the back-and-forth movement of the lancet holder 72 after the puncture may be effectively dampened.
In addition, if the puncture depth adjusting member 33 is moved back and forth, the second stab preventing rib 33b also goes back and forth and the timing of the braking application on the lancet holder changes, so the abutting stopping part 33c alone may be moved back and forth by rotating the puncture depth adjusting member 33 without going back and forth in this case.
Furthermore, even when there is no space for providing the rib member 80 formed on the inside of the housing 35 in Embodiment 2 described above, and effect the same as that in Embodiment 2 above may easily be obtained by just devising a shape for the rotating body 33, which is the existing puncture depth adjusting part. Furthermore, large increases in cost may be avoided by assuring ease of assembly without increasing the number of parts.

### INDUSTRIAL APPLICABILITY

Since the lancet device of the present invention has the effect of being able to effectively prevent multiple punctures, it may be used widely for lancet devices that incorporate two biasing parts oriented in opposition to each other and project and retract the puncture needle.

## Claims

1. A lancet device comprising:
a lancet holder where a lancet that includes a puncture needle is attached from the front side in the puncture direction and also has a colliding part that includes a surface that intersects the puncture direction,
a first biasing part that applies a force that moves the lancet holder forward in the puncture direction,
a second biasing part that retracts the lancet holder that has been moved forward by applying a force that is smaller than the force applied by the first biasing part, and
an abutting stopping member that has a collision receiving part that collides with the colliding part and limits the forward motion of the lancet holder in the puncture direction when the lancet holder receives the force applied by the first biasing part and moves forward in the puncture direction, and a contact part that comes into contact with the colliding part due to the elastic deformation arising when the colliding part and the collision receiving part collide.

2. The lancet device according to claim 1 wherein
the contact part of the abutting stopping member has a first contact part and a second contact part disposed so as to be opposed to each other along the puncture direction, and
when the colliding part passes between the first contact part and the second contact part and collides with the collision receiving part, the abutting stopping member elastically deforms, and the side surfaces of the colliding part are inserted between the first contact part and the second contact part.

3. The lancet device according to claim 1 or 2 wherein an elastic body is provided between the colliding part and the collision receiving part.

4. The lancet device according to any one of claims 1 through 3 wherein the first biasing part and the second biasing part are coil springs.

5. A lancet device comprising
a lancet holder has an elastic member that elastically deforms in a direction intersecting the puncture direction and includes a sloped surface sloped with respect to the puncture direction,
a first biasing part that applies a force that moves the lancet holder forward in the puncture direction,
a second biasing part that retracts the lancet holder that has moved forward by applying a force that is smaller than the force applied by the first biasing part, and
a pressing pressure part that comes into contact with the sloped surface on the elastic member and presses the elastic member in a direction intersecting the puncture direction when the lancet holder moves into the vicinity of the position of a state where the lancet holder can be projected by the first biasing part.

6. A lancet device comprising:
a lancet holder that has an elastic member that elastically deforms in a direction intersecting the puncture direction and includes a first sloped surface sloped with respect to the puncture direction and a second sloped surface with a slope angle larger than the first sloped surface,
a first biasing part that applies a force that moves the lancet holder forward in the puncture direction,
a second biasing part that retracts the lancet holder that has moved forward by applying a force that is smaller than the force applied by the first biasing part and
a pressing pressure part that comes into contact with the first sloped surface and the second sloped surface on the elastic member and presses the elastic member in a direction intersecting the puncture direction.

7. The lancet device according to claim 6 wherein
the first sloped surface is formed facing forward in the puncture direction and
the second sloped surface is formed facing backward in the puncture direction so as to be adjacent to the first sloped surface.

8. The lancet device according to claim 6 or 7 wherein
the pressing pressure part comes into contact with the first sloped surface and the second sloped surface when the lancet holder moves into the vicinity of the position of a state where the lancet holder can be projected by the first biasing part.

9. The lancet device according to any one of claims 5 through 8 wherein
the pressing pressure part is a rib member that is formed in a case part inside which the lancet holder is inserted and extends in a direction intersecting the puncture direction.

10. The lancet device according to any one of claims 5 through 8 wherein
the pressing pressure part is included in a puncture depth adjusting member that adjusts the puncture depth of a puncture needle attached to a tip of the lancet holder.

11. The lancet device according to any one of claims 5 through 8 wherein
the lancet holder has a contact part that contacts with an end portion of the second biasing part when the lancet holder is moved forward in the puncture direction.
